# EUROPÄISCHE PATENTSCHRIFT

(11) **EP 4 267 212 B1**
(45) Veröffentlichungstag und Bekanntmachung des Hinweises auf die Patenterteilung: **13.11.2024**
(21) Anmeldenummer: 21830929.2
(22) Anmeldetag: 22.12.2021
(51) Int. Cl.: A61L 31/02, A61L 31/14

(54) **VERFAHREN ZUR HERSTELLUNG EINES OBERFLÄCHENMODIFIZIERTEN METALLIMPLANTATS**
METHOD FOR PRODUCING A SURFACE-MODIFIED METAL IMPLANT
PROCÉDÉ DE PRODUCTION D'UN IMPLANT MÉTALLIQUE À SURFACE MODIFIÉE

(30) Priorität: 22.12.2020 AT 511222020
(43) Veröffentlichungstag der Anmeldung: 01.11.2023
(73) Patentinhaber: Johannes Kepler Universität Linz, 4040 Linz (AT)
(72) Erfinder: HEITZ, Johannes, 4040 Linz (AT); MUCK, Martina, 4040 Linz (AT); BAUMGARTNER, Werner, 4202 Kirchschlag (AT); MAIER, Christian, 8280 Fürstenfeld (AT); HASSEL, Achim Walter, 4209 Engerwitzdorf (AT)
(74) Vertreter: SONN Patentanwälte GmbH & Co KG
(86) Internationale Anmeldenummer: PCT/AT2021/060483
(87) Internationale Veröffentlichungsnummer: WO 2022/133509

(56) Entgegenhaltungen:
- HEITZ J ET AL: "Femtosecond laser-induced microstructures on Ti substrates for reduced cell adhesion", APPLIED PHYSICS A, SPRINGER BERLIN HEIDELBERG, BERLIN/HEIDELBERG, vol. 123, no. 12, 7 November 2017 (2017-11-07), pages 1 - 9, XP036355678, ISSN: 0947-8396, [retrieved on 20171107], DOI: 10.1007/S00339-017-1352-0

## Beschreibung

Die vorliegende Erfindung betrifft Verfahren zur Herstellung von oberflächenmodifizierten Metallimplantaten.

Metallimplantate finden vielfältige Anwendungen in der Medizin. Ein Anwendungsgebiet ist die Osteosynthese, welche beispielsweise in der Behandlung von Knochenbrüchen zum Einsatz kommt. Typischerweise werden bei der Osteosynthese zwei oder mehr Knochenfragmente operativ miteinander verbunden, mit dem Ziel, dass diese zusammenwachsen. Dabei können unterschiedliche Arten von Metallimplantaten eingesetzt werden, beispielsweise Knochenplatten, Schrauben, Nägel oder Drähte.

In vielen Fällen werden Metallimplantate nach der Verheilung wieder aus dem Körper des Patienten entfernt. Typischerweise kann die Verheilungszeit von Knochenbrüchen von 3 bis 15 Monaten betragen. Es ist klinische Praxis, die Entfernung so früh wie möglich nach erfolgter Fusionierung vorzunehmen, da das Operationsrisiko bei der Explantation steigt, je länger die Implantate im Körper verbleiben. Ein Grund dafür ist das Einwachsen des Implantats, was die Explantation erschwert. Explantationen werden daher oft zu einem Zeitpunkt vorgenommen, zu dem ein längerer Verbleib im Körper des Patienten aus therapeutischer Sicht noch von Nutzen gewesen wäre. In manchen Fällen werden Implantate sogar im Körper des Patienten belassen, beispielsweise wenn die möglichen schweren Komplikationen durch das verbleibende Implantat akzeptabel im Vergleich mit dem Operationsrisiko erscheinen, was speziell bei älteren Patienten der Fall sein kann.

Ist die Explantation nicht rechtzeitig erfolgt bzw. ein späteres Entfernen doch notwendig, kann es zu schwerwiegenden Komplikationen kommen. Beispielsweise können Schraubenköpfe von eingewachsenen Knochenschrauben abreißen. Die Entfernung der Schrauben ist dann nicht mehr minimal-invasiv möglich, sondern es muss offen operiert werden und es kann zur Beschädigung des Knochens kommen.

Zur Verhinderung des Einwachsens von Implantaten wurden zell-abweisende Beschichtungen vorgeschlagen, zum Beispiel Polymerschichten mit eingelagerten abweisenden Substanzen, zytotoxische Nano-Silber Schichten oder anti-adhäsive Polymere wie Polytetrafluorethylen (PTFE). Solche Beschichtungen gehen jedoch mit schwerwiegenden Nachteilen einher, unter anderem mangelnder Beständigkeit, der Notwendigkeit zusätzlicher Genehmigungen, unerwünschter Nebeneffekte oder der Notwendigkeit einer dauerhaften Einnahme von Medikamenten, falls die Beschichtungen in Kontakt mit Blut kommen.

Es besteht daher weiterhin ein Bedarf an Metallimplantaten mit reduziertem Einwachsverhalten, insbesondere reduziertem Einwachsen in das Knochengewebe. Es ist eine Aufgabe der Erfindung, solche Metallimplantate bzw. Verfahren zu deren Herstellung zur Verfügung zu stellen.

Die vorliegende Erfindung betrifft daher ein Verfahren zur Herstellung eines oberflächenmodifizierten Metallimplantats umfassend die folgenden Schritte:
(a) Bereitstellen eines Metallimplantats;
(b) Oxidieren einer Oberfläche des Metallimplantats zur Bildung einer oxidierten Oberfläche;
(c) Bestrahlen der oxidierten Oberfläche mit einem Ultrakurzpulslaser zur Erzeugung einer laserinduzierten Oberflächenstruktur.

In einem weiteren Aspekt betrifft die Erfindung ein oberflächenmodifiziertes Metallimplantat hergestellt durch das erfindungsgemäße Verfahren.

Im Zuge der Erfindung hat sich herausgestellt, dass das Oxidieren der Oberfläche des Implantats gefolgt von der Behandlung mit einem Ultrakurzpulslaser zu einem ausgeprägten zellabweisenden Effekt auf Osteoblasten führt. Osteoblasten sind Zellen, die für die Bildung von Knochengewebe beim Knochenumbau verantwortlich sind. Der durch das erfindungsgemäße Verfahren hervorgerufene Effekt auf diese Zellen ist daher von großem Vorteil für Implantate, die andernfalls in Knochengewebe einwachsen und nur noch schwer explantiert werden können.

Unterschiedliche Laserbehandlungen von Metalloberflächen sind aus dem Stand der Technik bekannt. Die KR 20110109272 A offenbart Oberflächenbehandlungen für Dentalimplantate, die zu einem aktivierenden Effekt auf Osteoblasten führen sollen. Die CN 110241451 B offenbart Verfahren zur Oberflächenmodifizierung von Titanimplantaten umfassend einen Oxidationsschritt gefolgt von einer Laserreinigung und dem Beschichten mit einer Nano-Hydroxyapatit-Suspension zur Verbesserung der Osseointegration. Die US 2006/0000814 A1 betrifft Verfahren zur Lasermarkierung und - texturierung von Materialien für die Mikroelektronik. Die EP 0475358 A1 betrifft implantierbare Prothesen mit sacklochartigen Vertiefungen, in welche Knochengewebe hineinwachsen kann. Die DE 10 2018 133 553 A1 Metallsubstrate mit Kanälen, in welche Lösungen oder Suspensionen aufgenommen werden können.

Aus dem Stand der Technik sind Verfahren zur Behandlung von Metallimplantaten bekannt, welche zu einem zellabweisenden Effekt auf Fibroblasten führen. Fibroblasten sind Zellen des Bindegewebes, welche beispielsweise eine Rolle beim unerwünschten Einwachsen von Herzschrittmachern spielen können. In diesem Zusammenhang offenbart Heitz et al. 2017 (Heitz, J., et al. "Femtosecond laser-induced microstructures on Ti substrates for reduced cell adhesion." Applied Physics A 123.12 (2017): 1-9) ein Verfahren umfassend eine Laserstrukturierung gefolgt von einer elektrochemischen Oxidation. Im Gegensatz zum erfindungsgemäßen Verfahren wird die Oxidation dabei nicht vor, sondern nach der Laserstrukturierung durchgeführt. Auf diese Weise behandelte Titanimplantate zeigen einen zellabweisenden Effekt gegen Fibroblasten. Ähnliche Verfahren zur Verminderung des Zellwachstums von Fibroblasten werden offenbart in Lone et al. 2020 ("Impact of femtosecond laser treatment accompanied with anodization of titanium alloy on fibroblast cell growth." Physica Status Solidi A 217 (2020): 1900838) und Fosodeder et al. 2020 ("Repellent rings at titanium cylinders against overgrowth by fibroblasts." Advanced Optical Technologies 9.3 (2020): 113-120)). Auch in diesen Fällen kommt ein Verfahren umfassend eine Laserstrukturierung gefolgt von einer elektrochemischen Oxidation zum Einsatz, was zu einem reduzierten Wachstum von Fibroblasten führt.

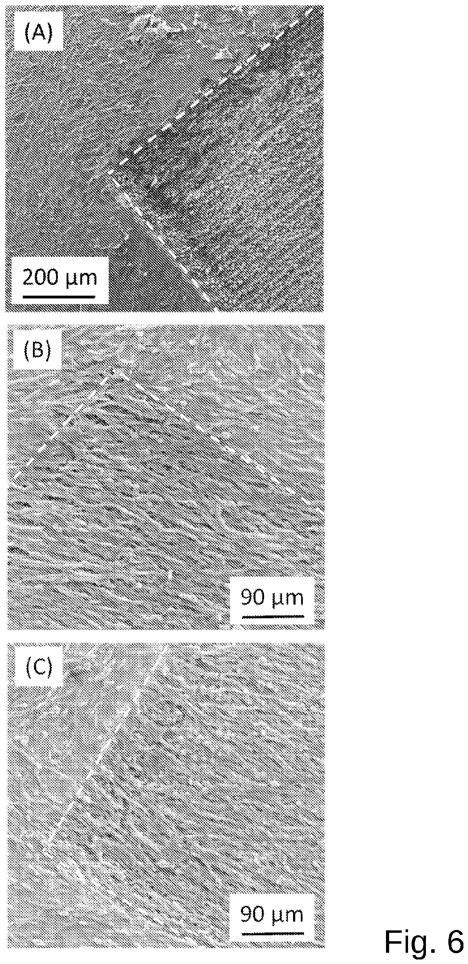

Im Rahmen der vorliegenden Erfindung hat sich gezeigt, dass die aus dem Stand der Technik bekannten Verfahren (Laserstrukturierung gefolgt von Oxidation) zu keinem zellabweisenden Effekt auf Osteoblasten führen (siehe Beispiel 3 sowie Figur 6B). Der bei Fibroblasten beobachtete Effekt tritt bei Osteoblasten nicht auf. Auch eine Laserbehandlung alleine führt zu keinem solchen Effekt. Überraschenderweise hat sich aber herausgestellt, dass eine Umkehr der Verfahrensschritte - eine Oxidation gefolgt von einer Laserstrukturierung - zu einem ausgeprägten zellabweisenden Effekt auf Osteoblasten führt (siehe Beispiel 3 sowie Figur 6A) .

Das erfindungsgemäße Verfahren ermöglicht es somit, Metallimplantate bereitzustellen, welche eine stark reduzierte Anhaftung von knochenbildenden Zellen aufweisen. Das Einwachsen in den Knochen (die sogenannte Osseointegration) wird verringert, was die Entfernung von Implantaten auch nach längeren Zeiträumen wesentlich erleichtert. Überraschenderweise ist die Reihenfolge der Behandlungsschritte entscheidend, um diesen Effekt zu erreichen. Eine Laserstrukturierung gefolgt von einer Oxidation (wie sie im Stand der Technik für Fibroblasten angewendet wird) sowie eine Laserstrukturierung ohne Oxidationsschritt bewirken den zellabweisenden Effekt im Gegensatz zum erfindungsgemäßen Verfahren nicht (siehe Beispiel 3 sowie Figur 6).

Nach Meinung der Erfinder - ohne hier an eine Theorie gebunden zu sein - lässt sich der beobachtete Effekt durch die Beschaffenheit der Oxidschicht auf der Metalloberfläche erklären. Die Laserbearbeitung einer Metalloberfläche mit bestehender Metalloxidschicht könnte zu einem ungleichmäßigen Abtrag der Oberfläche und somit zu einer inhomogenen Oxidschicht führen. Wird hingegen eine Oberflächenoxidierung nach der Laserbehandlung durchgeführt (wie aus dem Stand der Technik bekannt), könnte die Oxidierung die durch die Laserbehandlung entstandenen Inhomogenitäten ausgleichen. Nach Meinung der Erfinder ist die durch das erfindungsgemäße Verfahren erhältliche inhomogenere Metalloxidschicht für den Effekt auf das Zellwachstum von Osteoblasten verantwortlich.

In einer bevorzugten Ausführungsform weist die laserinduzierte Oberflächenstruktur eine laserinduzierte periodische Oberflächenstruktur (LIPSS) auf. Der Fachmann ist mit LIPSS sowie mit Verfahren zur Herstellung von LIPSS vertraut. Einen Überblick über LIPSS bieten z.B. Bonse 2016 ("Laser-induced periodic surface structures-A scientific evergreen." IEEE Journal of selected topics in quantum electronics 23.3 (2016)) sowie Schütz 2015 ("Gesteuerte Laserinduzierte Mikrostrukturen zur Beeinflussung des Reflexionsgrades von Halbleitern" Hannover: Gottfried Wilhelm Leibniz Universität Hannover, Dissertation, 2015). LIPSS werden ebenfalls in den hierin bereits zitierten Publikationen Heitz et al. 2017, Lone et al. 2020 und Fosodeder et al. 2020 beschrieben. LIPSS werden häufig auch als "Ripples" oder "Nanorippen" bezeichnet.

Im Zusammenhang mit der Erfindung hat es sich als besonders vorteilhaft erwiesen, wenn die LIPSS eine Periode zwischen 50 nm und 5 pm, vorzugsweise zwischen 65 nm und 2 pm, noch mehr bevorzugt zwischen 80 nm und 1 pm, insbesondere zwischen 100 nm und 500 nm aufweisen. Es hat sich gezeigt, dass solche Perioden für einen zellabweisenden Effekt besonders günstig sind. Vorzugsweise wird die Periode anhand von Rasterelektronenmikroskop (SEM)-Aufnahmen bestimmt, wobei der Abstand zwischen zehn aufeinanderfolgenden Tälern der Ripples vermessen und gemittelt wird.

Vorzugsweise weisen die LIPSS eine mittlere arithmetische Höhe Sa von zwischen 50 nm und 5 pm, vorzugsweise zwischen 65 nm und 2 pm, noch mehr bevorzugt zwischen 80 nm und 1 pm, insbesondere zwischen 100 nm und 500 nm auf. Die mittlere arithmetische Höhe Sa wird vorzugsweise bestimmt nach DIN EN ISO 25178-2:2012-09.

In einer weiteren bevorzugten Ausführungsform umfasst die laserinduzierte Oberflächenstruktur kegelartige Mikrostrukturen. Laserinduzierte kegelartige Mikrostrukturen werden häufig als "Cones" oder "Spikes" bezeichnet, welche typischerweise quasiperiodisch angeordnet sind. Der Fachmann ist mit solchen Strukturen und deren Herstellung vertraut. Neben den hierin bereits zitierten Publikationen Schütz 2015, Heitz et al. 2017, Lone et al. 2020 und Fosodeder et al. 2020 werden kegelartige Mikrostrukturen und deren Herstellung beispielsweise beschrieben in Fadeeva 2009 ("The hydrophobic properties of femtosecond laser fabricated spike structures and their effects on cell proliferation." Physica Status Solidi A 206.6 (2009): 1348-1351). Vorzugsweise beträgt die mittlere Strukturgröße der kegelartigen Mikrostrukturen zwischen 1 pm und 50 pm, bevorzugt zwischen 2 pm und 25 pm, noch mehr bevorzugt zwischen 3 µm und 15 µm. Die mittlere Strukturgröße der kegelartigen Mikrostrukturen kann beispielsweise anhand einer SEM-Aufnahme einer Fläche von 100 µm × 100 pm bestimmt werden, wobei die mittlere Strukturgröße als Mittelwert der Wurzeln der Flächeninhalte der kegelartigen Mikrostrukturen bestimmt werden kann (da jedes Strukturelement eine flächenförmige Ausdehnung und somit einen Flächeninhalt auf der Oberfläche aufweist).

Vorzugsweise weisen die kegelartigen Mikrostrukturen eine Spitzendichte zwischen 10³ und 10⁵ mm⁻² (Anzahl Spitzen pro Quadratmillimeter), bevorzugt zwischen 2×10³ und 5×10⁴ mm⁻², insbesondere zwischen 4×10³ und 2.5×10⁴ mm⁻² auf. Die Spitzendichte kann beispielsweise anhand einer SEM-Aufnahme einer Fläche von 100 µm × 100 pm bestimmt werden. Vorzugsweise wird die Spitzendichte bestimmt nach DIN EN ISO 25178-2:2012-09.

Vorzugsweise weisen die kegelartigen Mikrostrukturen eine mittlere arithmetische Höhe Sa von zwischen 1 pm und 50 pm, bevorzugt zwischen 2 pm und 25 pm, noch mehr bevorzugt zwischen 3 pm und 15 pm auf. Die mittlere arithmetische Höhe Sa wird vorzugsweise bestimmt nach DIN EN ISO 25178-2:2012-09.

Als besonders günstig hat es sich erwiesen, wenn die kegelartigen Mikrostrukturen von LIPSS überlagert sind. Im Rahmen der Erfindung haben beispielsweise auch LIPSS alleine (ohne kegelartige Mikrostrukturen) einen zellabweisenden Effekt, dieser wird durch das zusätzliche Vorliegen von kegelartigen Mikrostrukturen aber noch erheblich verstärkt.

In einer bevorzugten Ausführungsform weist die laserinduzierte Oberflächenstruktur eine hierarchische Oberflächenstruktur auf. Unter einer hierarchischen Oberflächenstruktur wird hierin eine Oberflächenstruktur verstanden, welche eine erste Strukturebene mit einer ersten mittleren arithmetische Höhe Sa₁, vorzugsweise zwischen 1 pm und 50 pm, bevorzugt zwischen 2 pm und 25 pm, noch mehr bevorzugt zwischen 3 µm und 15 pm, und eine die erste Strukturebene überlagernde zweite Strukturebene mit einer zweiten mittleren arithmetische Höhe Sa₂, vorzugsweise zwischen 50 nm und 5 pm, bevorzugt zwischen 65 nm und 2 pm, noch mehr bevorzugt zwischen 80 nm und 1 pm, insbesondere zwischen 100 nm und 500 nm, aufweist. Die mittleren arithmetischen Höhen Sa₁ und Sa₂ werden vorzugsweise bestimmt wie beschrieben in DIN EN ISO 25178-2:2012-09. In einer bevorzugten Ausführungsform beträgt das Verhältnis Sa₁:Sa₂ zwischen 200:1 und 2:1, vorzugsweise zwischen 10:1 und 5:1, noch mehr bevorzugt zwischen 50:1 und 10:1. Vorzugsweise weist die erste Strukturebene kegelartige Mikrostrukturen, wie hierin beschrieben, auf. Vorzugsweise weist die zweite Strukturebene LIPSS, wie hierin beschrieben, auf.

Der Fachmann ist mit der Erzeugung von laserinduzierten Oberflächenstrukturen mit einem Ultrakurzpulslaser vertraut. Geeignete Verfahren zur Oberflächenstrukturierung sind beispielsweise beschrieben in Heitz et al. 2017, Lone et al. 2020 und Fosodeder et al. 2020. Im Zusammenhang mit der Erfindung ist ein Ultrakurzpulslaser vorzugsweise eine Laserstrahlquelle, die gepulstes Laserlicht mit Pulslängen von weniger als 100 ps aussenden kann, vorzugsweise zwischen 0.1 fs und 100 ps. In einer bevorzugten Ausführungsform des erfindungsgemäßen Verfahrens wird die oxidierte Oberfläche mit einer Laserstrahlung mit einer Pulslänge von zwischen 0.1 fs und 100 ps, vorzugsweise zwischen 1 fs und 35 ps, noch mehr bevorzugt zwischen 10 fs und 1 ps bestrahlt.

In einer bevorzugten Ausführungsform wird die oxidierte Oberfläche mit einer Laserstrahlung bestrahlt, welche eine Wellenlänge zwischen 100 nm und 2000 nm, vorzugsweise zwischen 190 nm und 1600 nm, insbesondere zwischen 400 nm und 1100 nm, beispielsweise 800 nm oder 1064 nm, aufweist.

In einer bevorzugten Ausführungsform wird die oxidierte Oberfläche mit einer Laserstrahlung mit einer maximalen Laserfluenz von mindestens 0.1 J/cm², vorzugsweise mindestens 0.5 J/cm² bestrahlt. In einer weiteren bevorzugten Ausführungsform wird die oxidierte Oberfläche mit einer Laserstrahlung mit einer mittleren Laserfluenz von mindestens 0.05 J/cm², vorzugsweise mindestens 0.25 J/cm² bestrahlt. Die hierin als bevorzugt genannten Werte für die Pulslänge, Wellenlänge und Laserfluenz haben sich im Zusammenhang mit der Erfindung als besonders günstig für die Erzeugung von Oberflächenstrukturen erwiesen.

Das Oxidieren der Oberfläche kann durch gängige und dem Fachmann bekannte Oxidationsverfahren erfolgen. Das Oxidieren der Oberfläche führt vorzugsweise zur Bildung einer Oxidschicht auf der Oberfläche. Vorzugsweise erfolgt das Oxidieren der Oberfläche durch Anodisierung oder durch Flammenoxidation, insbesondere durch Anodisierung. Die Anodisierung ist eine gängige Vorbehandlung für Metallimplantate, die üblicherweise zur Verbesserung der Oberflächenbeständigkeit und zu Markierungszwecken dient. Im Rahmen des erfindungsgemäßen Verfahrens hat sich die Anodisierung als besonders vorteilhaft zum Erreichen des zellabweisenden Effekts auf Osteoblasten erwiesen. Ohne an eine Theorie gebunden zu sein ist die Anodisierung nach Ansicht der Erfinder überlegen, weil neben der Diffusion im Konzentrationsgradienten noch der thermisch aktivierte, feldunterstütze Sprung von Ionen genutzt wird die kinetische Barriere zum weiteren Oxidwachstum zu überwinden. Der Fachmann ist mit der Durchführung der Anodisierung von Metallimplantaten vertraut. Vorzugsweise wird die Anodisierung in einem sauren Elektrolyten durchgeführt, vorzugsweise Schwefelsäure oder Phosphorsäure, beispielsweise 0,1 mol/L Schwefelsäure. Die Anodisierung erfolgt vorzugsweise bei einer Anodisierungsspannung zwischen 1 und 150 V, vorzugsweise zwischen 2 und 50 V, insbesondere zwischen 5 und 20 V. Vorzugsweise kann die Potentialführung potentiodynamisch in Form einer linearen Potential-Zeit Rampe erfolgen oder durch potentiostatische Führung eines Sprungs auf das Endpotential oder galvanostatisch (technisch am einfachsten) bei der der Strom so lange konstant gehalten wird bis das Endpotential erreicht ist. Auch Kombinationen sind denkbar und üblich, bei denen zunächst galvanostatisch gearbeitet wird und dann nach Erreichen des Zielpotentials, letzteres noch eine gewisse Zeit potentiostatisch gehalten wird.

In einer bevorzugten Ausführungsform weist die Oberfläche eine Chrom-Cobalt-Molybdän-Legierung, einen Rostfreistahl (vorzugsweise Stähle die neben Eisen auch Chrom enthalten und dazu noch Nickel, Molybdän und weitere Legierungsbestandteile), Tantal, Titan oder eine Titan-Legierung auf, vorzugsweise Titan oder eine Titanlegierung. Vorzugsweise besteht die Oberfläche im Wesentlichen aus den genannten Materialien. Besonders bevorzugt ist es, dass das Metallimplantat im Wesentlichen aus den genannten Materialien besteht. Besonders bevorzugt sind Chrom-Cobalt-Molybdän-Legierungen ausgewählt aus ASTM F90 Co-20Cr-15W-10Ni, ASTM F562 Co-35Ni-20Cr-10Mo, oder ASTM F1537 Co-28Cr-6Mo; Stahl ausgewählt aus ASTM F138/F139 316L oder ASTM F1314 22Cr-13Ni-5Mn; Titan-Legierungen ausgewählt aus ASTM F67 unlegiertes Titan, ASTM F136 Ti-6Al-4V, oder ASTM F1295 Ti-6Al-7Nb; oder ASTM F560 unlegiertes Tantal. Insbesondere bevorzugt ist ASTM F67 unlegiertes Titan oder ASTM F136 Ti-6Al-4V.

Vorzugsweise ist das Metallimplantat ein medizinisches Metallimplantat. In einer bevorzugten Ausführungsform ist das Metallimplantat ein orthopädisches Metallimplantat, insbesondere ein Osteosynthese-Metallimplantat. Im Zusammenhang mit der Osteosynthese ist ein reduziertes Einwachsen des Implantats oft besonders vorteilhaft, da dies eine einfachere Explantation, beispielsweise nach dem Zusammenwachsen bzw. der Ausheilung eines Knochens, ermöglicht. In besonders bevorzugten Ausführungsformen ist das Osteosynthese-Metallimplantat eine Schraube, eine Platte, ein Nagel oder ein Draht.

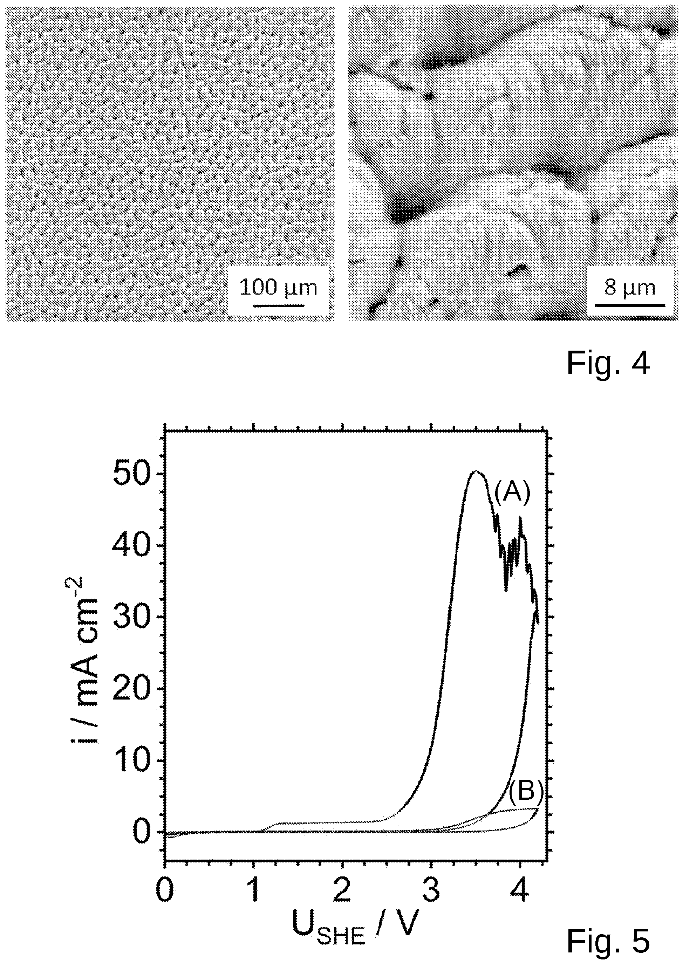

Die Reihenfolge der Verfahrensschritte des erfindungsgemäßen Verfahrens hat Auswirkungen auf die Beschaffenheit der Oberfläche des Metallimplantats. So hat sich gezeigt, dass die Oberfläche eines erfindungsgemäß hergestellten Metallimplantats in Cyclovoltammetriemessungen eine deutlich höhere Stromdichte aufweist als eine Oberfläche, welche zuerst laserbehandelt und danach oxidiert wurde (Umkehr der Verfahrensschritte; siehe Beispiel 2 sowie Figur 5). Nach Meinung der Erfinder lässt sich dies dadurch erklären, dass die Laserstrukturierung zu Inhomogenitäten der Oxidschicht auf der vergrößerten Oberfläche führt, wodurch ein größerer Anteil des Metalls im Bereich der Oberfläche nicht oxidiert ist und somit im Cylcovoltammetrieexperiment noch oxidiert werden kann. Erfolgt die Oxidation hingegen nach der Laserstrukturierung, liegt eine homogenere Oxidschicht vor (welche den ausgeprägten zellabweisenden Effekt auf Osteoblasten nicht aufweist) und der Anteil von nicht oxidiertem Metall im Bereich der Oberfläche ist geringer.

Vorzugsweise weist das Metallimplantat eine Stromdichte von zwischen 5 mA/cm² und 500 mA/cm², bevorzugt zwischen 10 mA/cm² und 200 mA/cm², noch mehr bevorzugt zwischen 20 mA/cm² und 100 mA/cm², insbesondere zwischen 30 mA/cm² und 80 mA/cm² auf, vorzugsweise gemessen durch Cyclovoltammetrie bei 3,5 V bei ansteigender Spannung mit einer Vorschubgeschwindigkeit von 0,1 V/s, einer Anfangsspannung von 0 V, einer Maximalspannung von 4,2 V, mit einer elektrochemischen Zelle bestehend aus der Oberfläche als Arbeitselektrode, einer Gold-Gegenelektrode und einer Ag/AgCl Elektrode in 3 M KCl als Referenzelektrode, bei 25 °C. Vorzugsweise wird die Stromdichte gemessen wie beschrieben in Beispiel 2.

Sämtliche hierin genannten Parameter beziehen sich, wenn nicht anders gekennzeichnet, auf SATP-Bedingungen nach IUPAC ("Standard Ambient Temperature and Pressure"), insbesondere auf eine Temperatur von 25 °C und einen Druck von 1013 hPa.

Die vorliegende Erfindung wird durch die folgenden Beispiele und die Figuren illustriert, auf welche sie selbstverständlich nicht eingeschränkt ist.

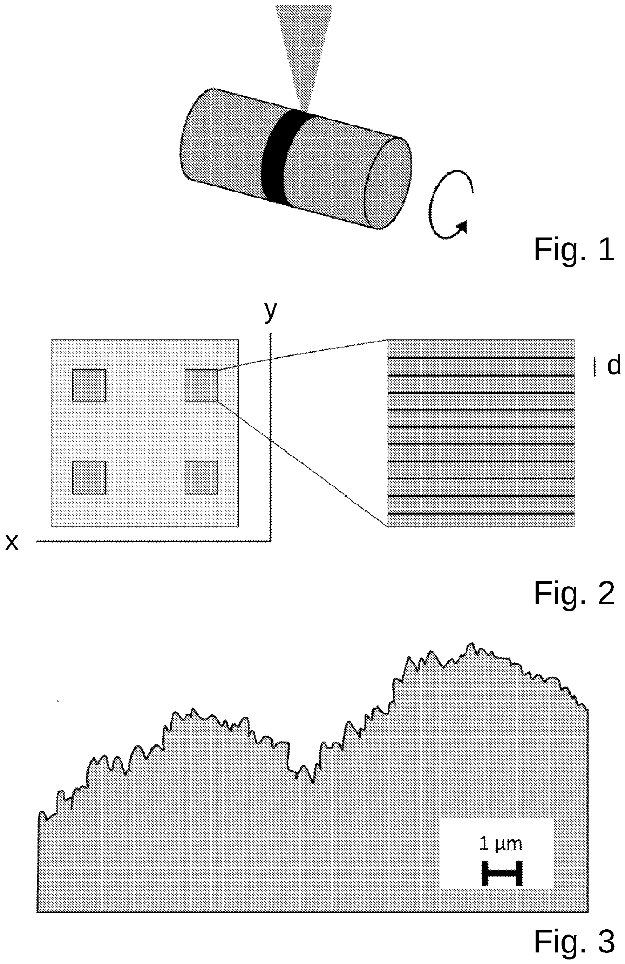

**Figur 1****:** Schematische Darstellung der Laserstrukturierung einer zylindrischen Probe. Die Probe wurde um ihre Symmetrieachse rotiert, während die Oberfläche mit einem Ultrakurzpulslaser bearbeitet wurde. Der Laserstrahl ist schematisch als Dreieck dargestellt; die bearbeitete Oberfläche ist schwarz dargestellt.
**Figur 2****:** Schematische Darstellung der Laserstrukturierung einer flachen Probe. Die Probe wies vier laserbearbeitete Flächen auf (dargestellt als dunklere Quadrate). Jede bearbeitete Fläche wurde Linie für Linie abgerastert, wodurch ein Mäandermuster mit Abstand d erzeugt wurde.
**Figur 3****:** Schematische Darstellung des Oberflächenprofils eines Metallimplantats aufweisend eine hierarchische Oberflächenstruktur, gemäß einer bevorzugten Ausführungsform der Erfindung. Das gezeigte Profil weist eine erste Strukturebene mit einer mittleren arithmetischen Höhe im Mikrometerbereich (z.B. kegelartige Mikrostrukturen) und eine zweite Strukturebene mit einer mittleren arithmetischen Höhe im Nanometerbereich (z.B. LIPSS) auf.
**Figur 4****:** Rasterelektronenmikroskop (SEM)-Aufnahmen der laserinduzierten Oberflächenstruktur eines erfindungsgemäß hergestellten Metallimplantats.
**Figur 5****:** Cyclovoltammetriemessungen von (A) einem Metallimplantat hergestellt nach dem erfindungsgemäßen Verfahren; und (B) einem Metallimplantat hergestellt nach dem gleichen Verfahren, wobei das Oxidieren der Oberfläche nach der Laserbehandlung erfolgte (Umkehr der Verfahrensschritte). Beim erfindungsgemäß hergestellten Metallimplantat wurde eine deutlich höhere Ladungsdichte gemessen.
**Figur 6****:** SEM-Aufnahmen des Wachstums von Osteoblasten auf Metallimplantaten, welche nach unterschiedlichen Verfahren hergestellt wurden. Die Grenze der Laser-bestrahlten Oberfläche ist jeweils durch eine weiße strichlierte Linie angezeigt. (A) Oxidation gefolgt Laserbehandlung (erfindungsgemäß): Der nicht laserbearbeitete Bereich war dicht mit konfluenten Lagen von Osteoblasten besiedelt, während der laserbearbeitete Bereich weitgehend zellfrei blieb. (B) Laserbehandlung gefolgt von Oxidation (Umkehr der Verfahrensschritte): Sowohl der laserbearbeitete als auch der nicht laserbearbeitete Bereich war dicht mit konfluenten Lagen von Osteoblasten besiedelt. (C) Laserbehandlung ohne Oxidation: Sowohl der laserbearbeitete als auch der nicht laserbearbeitete Bereich war dicht mit konfluenten Lagen von Osteoblasten besiedelt.

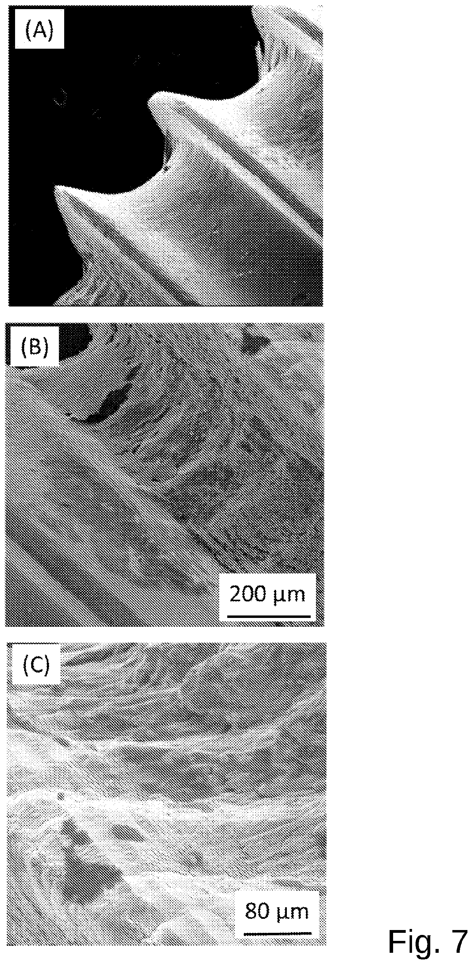

**Figur 7****:** SEM-Aufnahmen einer mit Osteoblasten bewachsenen Titan-Knochenschraube, hergestellt durch Anodisierung gefolgt von Bestrahlung mit einem Ultrakurzpulslaser. (A) Aufnahme eines nicht laserbearbeiteten Bereichs: Die Oberfläche war dicht mit konfluenten Lagen von Osteoblasten bedeckt. An den steilen Rändern der Schraubengänge hoben sich die Zellen teilweise ab, was typisch für knochenbildende Zellen ist. (B) Aufnahme eines laserbearbeiteten Bereichs: Nur wenige Osteoblasten waren sichtbar, große Bereiche blieben zellfrei. (C) Vergrößerung des laserbearbeiteten Bereichs: die dunklen Strukturen sind vereinzelte Osteoblasten.

### Beispiel 1: Herstellung von oberflächenmodifizierten Metallimplantaten

Als Proben wurden entweder flache F136 Ti-6Al-4V Proben der Firma Zapp Precision Metals GmbH (Schwerte, Deutschland) mit einer Fläche von 20 × 20 mm² oder anodisierte F136 Ti-6Al-4V Knochenschrauben oder Knochenplatten der Firma Hofer Medical Solutions (Fürstenfeld, Österreich) verwendet. Die Knochenschrauben oder Knochenplatten der Firma Hofer Medical Solutions hatten eine intensive blaue oder goldene Interferenzfarbe, was durch Anodisierung in einem sauren Elektrolyten (Anodisierbad aus 25-50 % Phosphorsäure) erreicht wurde. Die flachen F136 Ti-6Al-4V Proben wurden entweder vor (erfindungsgemäß) oder nach (Vergleichsversuche) der Laserbehandlung in 0,1 M Schwefelsäure bei einer Anodisierungsspannung von 10 V anodisiert, was zu einer braunen Interferenzfarbe führte.

Die Laserbehandlung wurde entweder mit einem verstärkten Femtosekunden Ti:Saphir Lasersystem (Hurricane-I, Spectra Physics, Darmstadt, Deutschland) mit einer Wellenlänge λ = 800 nm, Pulslänge τ = 120 fs, einer typischen Pulsenergie E = 50 pJ und einer Repetitionsrate von 1 kHz oder mit einem Yb-basierten verstärkten Femtosekunden-Lasersystem (Spirit 1040-16 HE, Spectra Physics, Darmstadt, Deutschland) mit einer Wellenlänge λ = 1040 nm, Pulslänge τ = 350 fs, einer typischen Pulsenergie E = 75 pJ und einer Repetitionsrate bis 200 kHz durchgeführt. Zwei lineare Verschiebeeinheiten wurden für die laterale Bewegung der Proben während der Bestrahlung eingesetzt. Zylindrische Proben, z.B. Knochenschrauben, wurden während der Bestrahlung mit einem dritten Schrittmotor oder Linearmotor um ihre Symmetrieachse rotiert (Figur 1). Flache Proben wurden Linie für Linie abgerastert (Figur 2). Der Laserstrahl wurde mit einer Linse mit einer Brennweite von f = 200 mm auf die Probe fokussiert, was dort zu einem Gauß-förmigen Strahlprofil mit einem Durchmesser von ungefähr 50 um bis 70 pm bezogen auf die Halbwertsbreite führte. Das Strahlprofil des Laserstrahls wurde mit einer digitalen Kamera ausgemessen. Die Bestrahlungsparameter (Geschwindigkeit des Strahls auf der Probe, Linienabstand, mittlere Energiedichte) wurden so gewählt, dass eine regulär geordnete Oberflächenstruktur bestehend aus etwa 10 bis 15 pm hohen kegelartigen Mikrostrukturen erreicht wurde, welche zusätzlich mit einer regulären Rippenstruktur ("Ripples", LIPSS) mit einer Periode von einigen 100 nm bedeckt waren. SEM-Aufnahmen der hergestellten laserinduzierten Oberflächenstrukturen sind in Figur 4 zu sehen. Von solchen Proben wurden auch Schnitte mit einem fokussierten Ionenstrahl angefertigt, woraus man Oberflächenprofile ermitteln kann, wie sie schematisch in Figur 3 dargestellt sind.

### Beispiel 2: Stromdichtemessungen an der Metallimplantatoberfläche durch Cyclovoltammetrie

Für die Cyclovoltammetrie an flachen Proben mit relativ kleinen laserbearbeiten Bereichen wurde eine standardisierte elektrochemische Zelle mit einer Arbeitselektrode (der zu messenden Probe), einer Gegenelektrode und einer Referenzelektrode benutzt, wie sie in Lone et al. 2020 beschrieben ist. Bei großflächigen oder zylindrischen Proben wurden die Proben direkt in den Elektrolyten getaucht. Bei der Messung wurden die Titan-Proben vollständig mit dem Elektrolyten (0,1 molare Schwefelsäure, belüftet) bedeckt und für 100 s bei offener Schaltkreispolarisation (englisch: open circuit polarization, OCP) belassen. Als Referenzelektrode wurde eine Ag/AgCl Elektrode in 3 molarer KCl Lösung benutzt, die ein elektrisches Potential von 0,195 V gegenüber einer Standardwasserstoffelektrode (englisch: standard hydrogen electrode, SHE) aufweist, und als Gegenelektrode ein Golddraht mit einer Fläche von etwa 7 cm². Der Elektrolyt wurde mittels eines Magnetrührers während der Messung ständig gerührt. Die Messung wurde bei Raumtemperatur durchgeführt. Für die Cyclovoltammetrie wurde die Spannung startend von 0 V mit einer Rate von ks = 0,1 V/s bis zur Maximalspannung (z.B. 4,2 V) erhöht und anschließend mit der gleichen Rate wieder auf 0 V abgesenkt. Als Messgröße wurde die Stromdichte i bezogen auf die laserbearbeite Fläche herangezogen, die gegen die Spannung U_{SHE} relativ zur Standardwasserstoffelektrode (SHE) aufgetragen wurde.

Figur 5 zeigt einen Vergleich von Cyclovoltammetrie-Messungen von einer nach dem erfindungsgemäßen Verfahren hergestellten flachen F136 Ti-6Al-4V Probe (A), d.h. sie wurde zuerst oxidiert und dann laserbearbeitet wie in Beispiel 1 beschrieben, und einer Probe (B), bei der diese Verfahrensschritte umgekehrt wurden. Beide Proben waren mit freiem Auge oder auch im Mikroskop nicht zu unterscheiden. Die Probe (A) zeigte aber ab einer Spannung von 2,5 bis 3 V eine stark ansteigende Stromdichte, die bei etwa 3,5 V ein lokales Maximum erreichte. Der Anstieg betrug dabei weit über 90 % der Maximalstromdichte. Dagegen verharrte die Stromdichte bei Probe (B) zwischen 2,5 und 3,5 V weitgehend auf einem niedrigen Niveau und zeigte keinen Anstieg um 90 % der Maximalstromdichte, die zudem weit unter der von Probe (A) lag.

### Beispiel 3: Wachstum von Osteoblasten auf flachen oberflächenmodifizierten Proben

Für die Zelltests wurden knochenbildende Zellen (Osteoblasten) der kommerziell erhältlichen humanen Zelllinie SAOS-2 verwendet. Die Zellen wurden in einem gängigen Zellmedium in mit Wasserdampf gesättigter Atmosphäre mit 5% CO₂ Anteil bei 37°C kultiviert und wurden einmal pro Woche im Verhältnis 1:10 geteilt. Die Experimente mit oxidierten und laserbearbeiten Proben sowie mit Vergleichsproben wurden mit typischen Besiedlungsdauern von 2 bis 3 Wochen durchgeführt. Nach einer Desinfektion mit Ethanol wurde jeweils ein Satz von Proben in eine Petrischale mit Zellen und Zellmedium gegeben, so dass die Proben vollständig bedeckt waren. Nach Ablauf der gewählten Besiedlungsdauer, während der die Petrischalen im Inkubator verblieben, wurden die Zellen auf den Proben fixiert und dehydratisiert. Danach wurden die Proben mit Gold besputtert und die Besiedlungsdichte mit Hilfe des Elektronenmikroskops ausgewertet.

Figur 6 zeigt Elektronenmikroskop-(SEM)-Aufnahmen von Osteoblasten nach einer Besiedlungsdauer von 2 Wochen auf teilweise laserbearbeiteten flachen F136 Ti-6Al-4V Proben. Bei Probe (A) handelt sich um eine Knochenplatte, die vor der Bearbeitung mit dem Femtosekundenlaser bei etwa 25 V Anodisierungsspannung anodisiert wurde, bei Probe (B) handelt sich um eine flache F136 Ti-6Al-4V Probe die zuerst mit dem Femtosekundenlaser bearbeitet wurde und dann mit 10 V Anodisierungsspannung anodisiert wurde und bei Probe (C) handelt es sich um eine flache F136 Ti-6Al-4V Probe die nur mit dem Femtosekundenlaser bearbeitet und nicht anodisiert wurde. Während bei allen drei Proben die nicht laserbearbeiteten Bereiche dicht mit konfluenten Lagen von Osteoblasten besiedelt waren, waren die laserbearbeiteten und zuvor anodisierten Bereiche auf Probe (A) weitgehend zellfrei und es war nur die laserinduzierte Oberflächenstruktur zu sehen. Dagegen waren die laserbearbeiteten Bereiche bei Probe (B) und (C) ebenfalls dicht mit Zellen bedeckt. Es zeigte sich hier aber eine Ausrichtung der Zellen (parallel zu den hier nicht sichtbaren Ripples), die in den nicht laserbearbeiteten Bereichen nicht zu sehen war.

Die Ergebnisse lassen sich wie folgt zusammenfassen:
- Oxidation gefolgt von Laserbearbeitung (erfindungsgemäß): starker zellabweisender Effekt (bearbeiteter Bereich in Fig. 6A) ;
- Laserbearbeitung gefolgt von Oxidation: kein zellabweisender Effekt (bearbeiteter Bereich in Fig. 6B);
- Laserbearbeitung ohne Oxidation: kein zellabweisender Effekt (bearbeiteter Bereich in Fig. 6C);
- Oxidation ohne Laserbearbeitung: kein zellabweisender Effekt (nicht bearbeiteter Bereich in Fig. 6A und 6B);
- weder Oxidation noch Laserbearbeitung: kein zellabweisender Effekt (nicht bearbeiteter Bereich in Fig. 6C).

### Beispiel 4: Wachstum von Osteoblasten auf Knochenschrauben

Osteoblasten-Besiedlungstests mit Knochenschrauben wurden durchgeführt wie beschrieben in Beispiel 3. Die anodisierten Knochenschrauben mit und ohne Laserbearbeitung wurden hergestellt wie beschrieben in Beispiel 1.

Figur 7 zeigt anhand von Elektronenmikroskop-(SEM)-Aufnahmen den Vergleich des Osteoblasten-Wachstums auf anodisierten Knochenschrauben ohne Laserbearbeitung (A) und mit Laserbearbeitung (B, C). Die Besiedlungsdauer war 3 Wochen. Probe (A) war dicht mit konfluenten Lagen von Osteoblasten bedeckt. An den steilen Rändern der Schraubengänge hoben sich die Zellen teilweise ab und wuchsen dreidimensional, was typisch für knochenbildende Zellen ist. Auf den laserbearbeiteten Bereichen (B, C) hatten sich viel weniger Osteoblasten angesiedelt und große Bereiche blieben zellfrei. Das ist speziell in der höheren Vergrößerung (C) zu sehen, wo man auch die laserinduzierten Strukturen (speziell die LIPSS) erkennen kann, die im Bild heller sind, während die wenigen Zellen dunkel erscheinen. Auch hier kam es teilweise zum Abheben der Zellen von der Oberfläche und einem dreidimensionalen Wachstum. Dies könnte für die Nutzung der Erfindung positiv sein, da somit die Verbindung der Schraube zur neugeformten Knochenmatrix schlechter wird und sich die Schraube leichter wieder entfernen lassen sollte.

## Patentansprüche

1. Verfahren zur Herstellung eines oberflächenmodifizierten Metallimplantats umfassend die folgenden Schritte:
(a) Bereitstellen eines Metallimplantats;
(b) Oxidieren einer Oberfläche des Metallimplantats zur Bildung einer oxidierten Oberfläche;
(c) Bestrahlen der oxidierten Oberfläche mit einem Ultrakurzpulslaser zur Erzeugung einer laserinduzierten Oberflächenstruktur aufweisend eine laserinduzierte periodische Oberflächenstruktur (LIPSS).

2. Verfahren nach Anspruch 1, **dadurch gekennzeichnet, dass** die LIPSS eine Periode zwischen 50 nm und 5 pm, vorzugsweise zwischen 65 nm und 2 pm, noch mehr bevorzugt zwischen 80 nm und 1 pm, insbesondere zwischen 100 nm und 500 nm aufweist.

3. Verfahren nach einem der Ansprüche 1 oder 2, **dadurch gekennzeichnet, dass** die laserinduzierte Oberflächenstruktur kegelartigen Mikrostrukturen umfasst.

4. Verfahren nach Anspruch 3, **dadurch gekennzeichnet, dass** die kegelartigen Mikrostrukturen von LIPSS überlagert sind.

5. Verfahren nach Anspruch 1, **dadurch gekennzeichnet, dass** das Oxidieren der Oberfläche durch Anodisierung erfolgt.

6. Verfahren nach einem der Ansprüche 1 bis 5, **dadurch gekennzeichnet, dass** die oxidierte Oberfläche mit einer Laserstrahlung mit einer Pulslänge von zwischen 0.1 fs und 100 ps, vorzugsweise zwischen 1 fs und 35 ps, noch mehr bevorzugt zwischen 10 fs und 1 ps bestrahlt wird.

7. Verfahren nach einem der Ansprüche 1 bis 6, **dadurch gekennzeichnet, dass** die oxidierte Oberfläche mit einer Laserstrahlung bestrahlt wird, welche eine Wellenlänge zwischen 100 nm und 2000 nm, vorzugsweise zwischen 190 nm und 1600 nm, insbesondere zwischen 400 nm und 1100 nm aufweist.

8. Verfahren nach einem der Ansprüche 1 bis 7, **dadurch gekennzeichnet, dass** die oxidierte Oberfläche mit einer Laserstrahlung mit einer maximalen Laserfluenz von mindestens 0.1 J/cm², vorzugsweise mindestens 0.5 J/cm² bestrahlt wird.

9. Verfahren nach einem der Ansprüche 1 bis 8, **dadurch gekennzeichnet, dass** die oxidierte Oberfläche mit einer Laserstrahlung mit einer mittleren Laserfluenz von mindestens 0.05 J/cm², vorzugsweise mindestens 0.25 J/cm² bestrahlt wird.

10. Verfahren nach einem der Ansprüche 1 bis 9, **dadurch gekennzeichnet, dass** die Oberfläche Titan oder eine Titanlegierung aufweist, vorzugsweise im Wesentlichen daraus besteht.

11. Verfahren nach einem der Ansprüche 1 bis 10, **dadurch gekennzeichnet, dass** das Metallimplantat ein medizinisches Metallimplantat ist, bevorzugt ein orthopädisches Metallimplantat, besonders bevorzugt ein Osteosynthese-Metallimplantat, insbesondere eine Schraube, eine Platte, ein Nagel oder ein Draht.

12. Oberflächenmodifiziertes Metallimplantat hergestellt durch das Verfahren gemäß einem der Ansprüche 1 bis 11.

## Claims

1. Method for producing a surface-modified metal implant, comprising the following steps:
(a) providing a metal implant;
(b) oxidising a surface of the metal implant to form an oxidised surface;
(c) irradiating the oxidised surface using an ultrashort pulse laser to generate a laser-induced surface structure having a laser-induced periodic surface structure (LIPSS).

2. Method according to claim 1, **characterised in that** the LIPSS has a period between 50 nm and 5 µm, preferably between 65 nm and 2 µm, even more preferably between 80 nm and 1 µm, in particular between 100 nm and 500 nm.

3. Method according to either of claims 1 or 2, **characterised in that** the laser-induced surface structure comprises cone-like microstructures.

4. Method according to claim 3, **characterised in that** the cone-like microstructures are superimposed by LIPSS.

5. Method according to claim 1, **characterised in that** the surface is oxidised by means of anodisation.

6. Method according to any of claims 1 to 5, **characterised in that** the oxidised surface is irradiated using laser radiation which has a pulse length of between 0.1 fs and 100 ps, preferably between 1 fs and 35 ps, even more preferably between 10 fs and 1 ps.

7. Method according to any of claims 1 to 6, **characterised in that** the oxidised surface is irradiated using laser radiation which has a wavelength between 100 nm and 2000 nm, preferably between 190 nm and 1600 nm, particularly between 400 nm and 1100 nm.

8. Method according to any of claims 1 to 7, **characterised in that** the oxidised surface is irradiated using laser radiation which has a maximum laser fluence of at least 0.1 J/cm², preferably at least 0.5 J/cm².

9. Method according to any of claims 1 to 8, **characterised in that** the oxidised surface is irradiated using laser radiation which has an average laser fluence of at least 0.05 J/cm², preferably at least 0.25 J/cm².

10. Method according to any of claims 1 to 9, **characterised in that** the surface comprises titanium or a titanium alloy, preferably consists substantially thereof.

11. Method according to any of claims 1 to 10, **characterised in that** the metal implant is a medical metal implant, preferably an orthopaedic metal implant, particularly preferably an osteosynthesis metal implant, in particular a screw, a plate, a nail or a wire.

12. Surface-modified metal implant produced by means of the method according to any of claims 1 to 11.

## Revendications

1. Procédé de fabrication d'un implant métallique à surface modifiée, comprenant les étapes suivantes :
(a) la préparation d'un implant métallique ;
(b) l'oxydation d'une surface de l'implant métallique pour former une surface oxydée ;
(c) l'irradiation de la surface oxydée avec un laser à impulsion ultracourte pour produire une structure de surface induite par laser présentant une structure de surface périodique induite par laser (LIPSS).

2. Procédé selon la revendication 1, **caractérisé en ce que** la LIPSS présente une période comprise entre 50 nm et 5 µm, de préférence entre 65 nm et 2 µm, de manière encore mieux préférée entre 80 nm et 1 µm, en particulier entre 100 nm et 500 nm.

3. Procédé selon l'une des revendications 1 ou 2, **caractérisé en ce que** la structure de surface induite par laser comprend des microstructures en forme de cône.

4. Procédé selon la revendication 3, **caractérisé en ce que** les microstructures en forme de cône sont recouvertes de LIPSS.

5. Procédé selon la revendication 1, **caractérisé en ce que** l'oxydation de la surface s'effectue par anodisation.

6. Procédé selon l'une des revendications 1 à 5, **caractérisé en ce que** la surface oxydée est irradiée par un rayon laser ayant une longueur d'impulsion comprise entre 0,1 fs et 100 ps, de préférence entre 1 fs et 35 ps, de manière encore mieux préférée entre 10 fs et 1 ps.

7. Procédé selon l'une des revendications 1 à 6, **caractérisé en ce que** la surface oxydée est irradiée par un rayon laser qui présente une longueur d'onde comprise entre 100 nm et 2000 nm, de préférence entre 190 nm et 1600 nm, en particulier entre 400 nm et 1100 nm.

8. Procédé selon l'une des revendications 1 à 7, **caractérisé en ce que** la surface oxydée est irradiée par un rayon laser ayant une fluence laser maximale d'au moins 0,1 J/cm², de préférence d'au moins 0,5 J/cm².

9. Procédé selon l'une des revendications 1 à 8, **caractérisé en ce que** la surface oxydée est irradiée par un rayon laser ayant une fluence laser moyenne d'au moins 0,05 J/cm², de préférence d'au moins 0,25 J/cm².

10. Procédé selon l'une des revendications 1 à 9, **caractérisé en ce que** la surface présente du titane ou un alliage de titane, de préférence s'en compose essentiellement.

11. Procédé selon l'une des revendications 1 à 10, **caractérisé en ce que** l'implant métallique est un implant métallique médical, de préférence un implant métallique orthopédique, de manière particulièrement préférée un implant métallique d'ostéosynthèse, en particulier une vis, une plaque, un clou ou un fil.

12. Implant métallique à surface modifiée fabriqué par le procédé selon l'une des revendications 1 à 11.
